(19) Europäisches Patentamt
European Patent Office
Office européen des brevets

(11) Veröffentlichungsnummer: **0 239 874 A2**

(12) **EUROPÄISCHE PATENTANMELDUNG**

(21) Anmeldenummer: **87103922.8**

(22) Anmeldetag: **18.03.87**

(51) Int. Cl.4: **C07K 5/02**, C07D 233/64, C07K 1/06, A61K 37/64

(30) Priorität: **29.03.86 DE 3610593**

(43) Veröffentlichungstag der Anmeldung: **07.10.87 Patentblatt 87/41**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Wegmann, Helmut, Dr.**
**Wilhelm-Leuschner Strasse 4**
**D-6238 Hofheim am Taunus(DE)**
Erfinder: **Wagner, Adalbert, Dr.**
**Kleiststrasse 9**
**D-6238 Hofheim am Taunus(DE)**
Erfinder: **Kleemann, Heinz-Werner, Dr.**
**Jahnstrasse 6**
**D-6092 Kelsterbach(DE)**
Erfinder: **Ruppert, Dieter, Dr.**
**Patrizierhof 1**
**D-6233 Kelkheim (Taunus)(DE)**

(54) **Substituierte 4-Amino-3-hydroxybuttersäure-Derivate, Verfahren zu deren Herstellung, diese enthaltende Mittel und ihre Verwendung.**

(57) Die Erfindung betrifft Verbindungen der Formel

$$R^1\text{-A-B-NH-}\underset{|}{\overset{R^2}{CH}}\text{-}\overset{OH}{CH}\text{-}\overset{R^3}{CH}\text{-}\overset{O}{\overset{\|}{C}}\text{-}R^4$$

worin $R^1$ abwesend ist oder Wasserstoff, Alkyl oder Acyl, A einen Carbonsäurerest oder einen Aminosäurerest, B einen Aminosäurerest bedeuten und $R^2$, $R^3$ und $R^4$ wie in der Beschreibung definiert sind, sowie deren Salze, Verfahren zu ihrer Herstellung, diese enthaltende pharmazeutische Präparate und deren Verwendung als Arzneimittel sowie Zwischenprodukte zur Herstellung dieser Verbindungen.

EP 0 239 874 A2

Xerox Copy Centre

## Substituierte 4-Amino-3-hydroxybuttersäure-Derivate, Verfahren zu deren Herstellung, diese enthaltende Mittel und ihre Verwendung

Aus der EP-A-152 255 und der EP-A-155 809 sind Tripeptid-Derivate und deren Verwendung als Renin-Inhibitoren bekannt.

Es wurden neue substituierte 4-Amino-3-hydroxybuttersäure-Derivate gefunden, die in vitro und in vivo hochwirksam das Enzym Renin hemmen.

Die Erfindung betrifft Verbindungen der Formel I

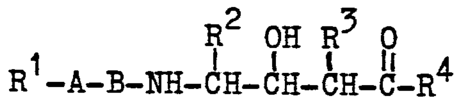

(I)

in welcher

$R^4$ $a_1$) einen Rest der Formel II bedeutet,

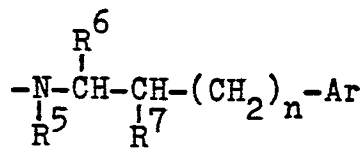

(II)

worin $R^5$ für Wasserstoff oder ($C_1$-$C_7$)-Alkyl und

$R^6$ für Wasserstoff, ($C_1$-$C_7$)-Alkyl oder durch Hydroxy, ($C_1$-$C_5$)-Alkoxy, ($C_1$-$C_5$)-Alkylthio, Carboxy, ($C_1$-$C_5$)-Alkoxycarbonyl, Cl, Br, ($C_1$-$C_5$)-Alkylamino, Di-($C_1$-$C_5$)-alkylamino, ($C_1$-$C_5$)-Alkoxycarbonylamino oder ($C_1$-$C_{15}$)-Aralkyloxycarbonylamino monosubstituiertes ($C_1$-$C_7$)-Alkyl stehen,

$R^7$ $a_1$) falls A wie unten unter ($c_2$) definiert ist, OH bedeutet,

$a_2$) andernfalls wie $R^6$ definiert ist oder OH bedeutet,

n 0, 1, 2, 3, oder 4 ist und

Ar für ($C_6$-$C_{14}$)-Aryl, das gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkoxy, ($C_1$-$C_7$)-Alkoxycarbonyl, Amino, ($C_1$-$C_7$)-Alkylamino, Di-($C_1$-$C_7$)-Alkylamino, Carboxy, Carboxymethyloxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo und Guanidinomethyl substituiert ist, oder für den Rest eines 5-oder 6-gliedrigen monocyclischen oder 9-oder 10-gliedrigen bicyclischen Heteroaromaten mit mindestens 1 C-Atom, 1 - 3 N-Atomen und/oder 1 S-oder O-Atom als Ringglieder steht, wobei Ar auch über -O-gebunden sein kann,

$R^1$ $b_1$) abwesend ist oder Wasserstoff bedeutet,

$b_2$) ($C_1$-$C_{20}$)-Alkyl, das gegebenenfalls durch ein, zwei oder drei gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, ($C_1$-$C_8$)-Alkanoyloxy, Cl, Br, Amino, ($C_1$-$C_7$)-Alkylamino, Di-($C_1$-$C_7$)-alkylamino, ($C_1$-$C_5$)-Alkoxycarbonylamino oder ($C_7$-$C_{11}$)-Aralkyloxycarbonylamino substituiert ist,

($C_3$-$C_8$)-Cycloalkyl,

($C_3$-$C_8$)-Cycloalkyl-($C_1$-$C_{10}$)alkyl oder

($C_6$-$C_{14}$)-Aryl-($C_1$-$C_8$)-alkyl, das im Arylteil gegebenenfalls durch einen oder zwei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist, bedeutet oder

$b_3$) einen Rest der Formel III bedeutet,

$R^a$-W-(III)

worin W für -CO-, -O-CO-, $-SO_2$-oder -NH-CO-steht und $R^a$ Wasserstoff, ($C_1$-$C_{10}$)-Alkyl, das gegebenenfalls ein-oder zweifach ungesättigt ist und das gegebenenfalls durch bis zu 3 gleiche oder verschiedene Rest aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-Alkylamino, ($C_1$-$C_5$)-Alkoxycarbonylamino, ($C_7$-$C_{15}$)-Aralkoxycarbonylamino oder 9-Fluorenylmethyloxycarbonylamino monosubstituiert ist,

($C_3$-$C_8$)-Cycloalkyl,

($C_3$-$C_8$)-Cycloalkyl-($C_1$-$C_6$)alkyl, ($C_6$-$C_{14}$)-Aryl, das gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl,

Amino, gegebenenfalls mit bis zu 2 Halogen substituiertes Anilino und Trifluormethyl substituiert ist, $(C_6\text{-}C_{14})$-Aryl-$(C_1\text{-}C_6)$-alkyl, worin der Arylteil gegebenenfalls wie vorstehend bei Aryl definiert ist, Heteroaryl oder Heteroaryl-$(C_1\text{-}C_6)$-alkyl, wobei der Rest des Heteroaromaten jeweils wie Ar bei $R^4$ definiert ist, bedeutet,

A $c_1$) einen Rest der Formel IV bedeutet,

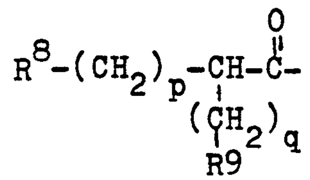 (IV)

in welcher p und q gleich oder verschieden sind und 0, 1, 2, 3 oder 4 bedeuten, $R^8$ und $R^9$ gleich oder verschieden sind und Phenyl, 2-oder 3-Thienyl, 2-, 3-oder 4-Pyridyl, 1-, 2-oder 4-Imidazolyl, OH oder Wasserstoff bedeuten oder

$c_2$) einen N-terminal mit $R^1$ und C-terminal mit B verknüpften Rest eine Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, 0-Methyltyrosin, 0-Benzyltyrosin, 0-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Norleucin, 1,2,3,4-Tetrahydroisochinolin-3-carbonsäure, Homophenylalanin, DOPA, 0-Dimethyldopa, 2-Amino-4-(2-thienyl-buttersäure, 2-Amino-4-(3-thienyl)-buttersäure, 3-(2-Thienyl)-serin, (Z)-Dehydrophenylalanin und (E)-Dehydrophenylalanin bedeutet,

B einen Rest einer Aminosäure, wie unter ($c_2$) definiert, bedeutet,

$R^2$ Wasserstoff, $(C_1\text{-}C_{10})$-Alkyl, $(C_4\text{-}C_7)$-Cycloalkyl, $(C_4\text{-}C_7)$-Cycloalkyl-$(C_1\text{-}C_4)$-alkyl, $(C_6\text{-}C_{14})$-Aryl oder $(C_6\text{-}C_{14})$-Aryl-$(C_1\text{-}C_4)$-alkyl bedeutet und

$R^3$ Wasserstoff, $(C_1\text{-}C_{10})$-Alkyl, $(C_6\text{-}C_{14})$-Aryl oder $(C_6\text{-}C_{14})$-Aryl-$(C_1\text{-}C_4)$-alkyl bedeutet,

sowie deren physiologisch verträglichen Salze.

Die durch $R^2$, Hydroxy und $R^3$ substituierten C-Atome können jeweils die R-, S-oder R,S-Konfiguration besitzen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

$(C_6\text{-}C_{14})$-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Bevorzugte Aralkylreste sind Benzyl und Phenethyl.

Unter einem Rest eines 5-oder 6-gliedrigen monocyclischen oder 9-oder 10-gliedrigen bicyclischen Heteroaromaten mit mindestens 1 C-Atom, 1 - 3 N-Atomen und/oder 1 S-oder O-Atom als Ringglieder werden Reste von Heteroaromaten verstanden, wie sie beispielsweise in Katritzky, Lagowski, Chemie der Heterocyclen, Berlin, Heidelberg 1968, Seite 3 - 5 definiert sind. Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin. Entsprechendes gilt für von Heteroaryl abgeleitete Reste, wie z.B. Heteroaryloxy, Heteroaroyl und Heteroarylalkyl.

Die Aminosäuren A und B in Formel I sind sind durch eine Amidbindung miteinander verknüpft, es handelt sich um natürliche oder nichtnatürliche α-Aminosäuren der L-, D-oder D,L Konfiguration, vorzugsweise der L-Konfiguration. Entsprechendes gilt für Aminosäuren oder deren Derivate als Reste $R^4$.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali-oder Erdalkalimetallen gebildet, wie Na, K, Mg und Ca, sowie mit physiologich verträglichen organischen Aminen, wie z.B. Triethylamin und Tri-(2-hydroxy)-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon-oder Sulfonsäure, wie z.B. Essigsäure, Citronensäure, Benzoesäure, Maleinsäure, Fumarsäure, Weinsäure und p-Toluolsulfonsäure.

Bevorzugt sind Verbindungen der Formel I,
in welcher
$R^5$ Wasserstoff oder Methyl und/oder
$R^6$ Wasserstoff, Methyl, Ethyl, Propyl, Isopropyl, Butyl, Isobutyl, sec. Butyl, $(C_1-C_5)$-Alkoxycarbonylmethyl, $(C_1-C_5)$-Alkoxycarbonylethyl oder Methylthioethyl bedeuten und/oder worin
$R^7$ Wasserstoff falls A wie unter $(c_2)$ definiert oder OH bedeutet und/oder worin
Ar für Phenoxy, Phenyl-, 2-, 3-oder 4-Pyridyl, 4-Imidazolyl, 1-, 3-oder 4-Isochinolyl, 2-, 3-oder 4-Carboxyphenyl, 2-, 3-oder 4-$(C_1-C_7)$-Alkoxycarbonylphenyl, vorzugsweise 3-Methoxycarbonylphenyl oder 3-tert.-Butoxycarbonylphenyl, 2-, 3-oder 4-Aminophenyl, Mono-oder Di-$(C_1-C_7)$-alkylaminophenyl, vorzugsweise 3-Methylaminophenyl oder 3-Dimethylaminophenyl, 2-, 3-oder 4-Carbamoylphenyl, 3-Sulfamoylphenyl, 3-Sulfophenyl, Amino-$(C_1-C_7)$-alkylphenyl, vorzugsweise 2-3-oder 4-Aminomethylphenyl, 3-(Methylamino-methyl)-phenyl, 3-(N,N-Dimethylaminomethyl)-phenyl, $(C_1-C_7)$-Alkoxycarbonylmethoxyphenyl, vorzugsweise 2-, 3-oder 4-Methoxycarbonylmethyoxyphenyl oder 3-tert.-Butoxycarbonylmethoxyphenyl, 2-, 3-oder 4-Carboxymethyloxyphenyl, $(C_1-C_7)$-Alkoxyphenyl, vorzugsweise 2-, 3-oder 4-Methoxyphenyl, oder 2-, 3-oder 4-Guanidinomethylphenyl steht.

$R^1$ ist vorzugsweise abwesend, bedeutet Wasserstoff oder steht für $(C_1-C_{10})$-Alkyl, Cyclopentyl, Cyclohexyl, Cyclopentyl-$(C_1-C_{10})$-alkyl, Cyclohexyl-$(C_1-C_{10})$-alkyl, gegebenenfalls substitutiertes Phenyl-$(C_1-C_8)$-alkyl, $H_2N$-$(C_1-C_{10})$-Alkyl, HO-$(C_1-C_{10})$-Alkyl, $(C_1-C_4)$-Alkoxy-$(C_1-C_{10})$-alkyl, $(C_1-C_4)$-Alkoxycarbonyl-$(C_1-C_{10})$-alkyl, Carboxy-$(C_1-C_{10})$-alkyl, $(C_1-C_8)$-Alkanoyloxy-$(C_1-C_{10})$-alkyl, $(C_1-C_{11})$-Alkanoyl, wie n-Decanoyl, Formyl, Acetyl, Pivaloyl, Isovaleryl oder Isobutyryl, gegebenenfalls geschütztes Amino-$(C_1-C_{11})$-alkanoyl, wie 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl, Di-$(C_1-C_7)$-alkylamino-$(C_1-C_{11})$-alkanoyl, wie Dimethylaminoacetyl, $(C_4-C_9)$-Cycloalkanoyl, wie Cyclopropanoyl, Cyclobutanoyl, Cyclopentanoyl oder Cyclohexanoyl, $(C_6-C_{10})$-Aryl-$(C_1-C_{11})$-alkanoyl, wie Phenylacetyl, Phenylpropanoyl oder Phenylbutanoyl, 2-(o,o-Dichloroanilino)-phenylacetyl, 2-(N-Benzyl-o,o-dichloranilion)-phenylacetyl, gegebenenfalls durch Halogen, $(C_1-C_7)$-Alkyl, $(C_1-C_7)$-Alkoxy oder $(C_1-C_7)$-Alkoxycarbonyl substituiertes Benzoyl, wie 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzylsulfonyl, $(C_1-C_{10})$-Alkoxycarbonyl, wie Methoxycarbonyl oder tert.-Butoxycarbonyl, durch Halogen substitutiertes $(C_1-C_{10})$-Alkoxycarbonyl, wie 2,2,2-Trichlorethoxycarbonyl oder 1,1-Dimethyl-2,2,2-trichlorethoxycarbonyl, $(C_6-C_{14})$-Aryl-$(C_1-C_6)$-alkoxycarbonyl, wie Benzyloxycarbonyl oder 9-Fluorenylmethylcarbonyl, (2S)-trans-4-Hydroxy-1-acetylpyrrolidinyl-2-carbonyl, α-Methoxycarbonyl-α-benzyloxycarbonylaminoacetyl. N-Acetyl-(2S,3aS,6aS)-octahydrocyclopenta[b]pyrrol-2-carbonyl oder N-Acetyl-(2R,3aR,6aR)-octahydrocyclopenta[b]pyrrol-2-carbonyl.

Bevorzugte Aminosäuren, die für die Reste A, B und $R^4$ in Frage kommen, sind Phenylalanin, Histidin, Tyrosin, Tryptophan, Methionin, Leucin, Isoleucin, Asparagin, Asparaginsäure, β-2-Thienylalanin, β-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, 0-tert.-Butyltyrosin, Phenylglycin, 1-Naphthylalanin, 2-Naphtylalanin, 4-Nitrophenylalanin, Norleucin, 1,2,3,4-Tetrahydroisochinolin-3-carbonsäure, Homophenylalanin, 2-Amino-4-(2-thienyl)-buttersäure, (Z)-Dehydrophenylalanin oder (E)-Dehydrophenylalanin. Daneben bedeutet A vorzugsweise einen Rest der Formel IV wie unter $(c_1)$ beschrieben.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihre physiologisch verträgliches Salz überführt.

Fragmente einer Verbindung der Formel I mit einer endständigen Carboxylgruppe besitzen die nachstehenden Formeln Va - Vd:
$R^1$-OH (Va)
$R^1$-A-OH (Vb)
$R^1$-A-B-OH (Vc)

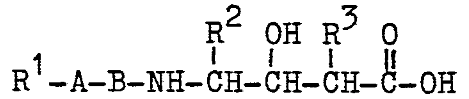
(Vd)

Fragmente einer Verbindung der Formel I mit einer endständigen Aminogruppe besitzen die nachstehenden Formeln VIa - VId:

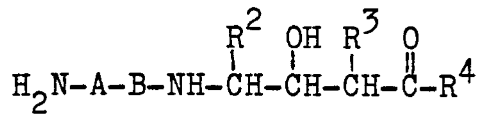 (VIa)

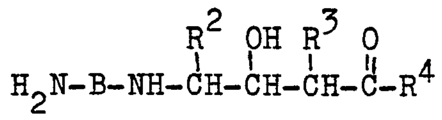 (VIb)

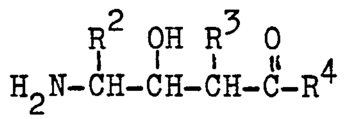 (VIc)

$H_2N-R^4$ (VId)

Methoden, die zur Herstellung einer Amidbindung geeignet sind, werden z.B. in Houben-Weyl, Methoden der organischen Chemie, Band 15/2 beschrieben, vorzugsweise werden die folgenden Methoden herangezogen; Aktivestermethode mit N-Hydroxy-succinmid als Esterkomponente, Kopplung mit Propanphosphonsäureanhydrid und die Gemischt-Anhydrid-Methode mit Pivaloylchlorid.

Die Herstellung der als Ausgangsverbindung verwendeten optisch aktiven Amine der Formel VII

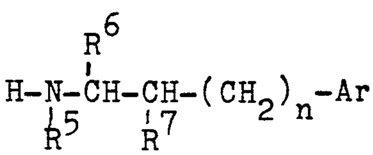 (VII)

worin n, $R^5$, $R^6$, $R^7$ und Ar wie oben definiert sind, erfolgt ausgehend von optisch aktiven α-Aminosäuren, wobei deren Asymmetriezentrum erhalten bleibt. Hierzu wird in bekannter Weise ein N-geschützter Aminosäurealdehyd hergestellt, welcher in einer Aldol-analogen Addition an einen entsprechenden Arylalkyl-Baustein gekoppelt nach Abspaltung der N-Schutzgruppe Aminoalkohole der Formel VII mit $R^7$ = OH ergibt. Man erhält Diastereomerengemische bezüglich des $R^7$-tragenden Zentrums, die in an sich bekannter Weise, beispielsweise durch fraktionierte Kristallisation oder durch Chromatographie getrennt werden.

Die Überprüfung der Diastereomerenreinheit erfolgt mittels HPLC, die Enantiomerenreinheit kann in bekannter Weise durch Überführung in Mosher-Derivate überprüft werden (H.S. Mosher et. al. J. org. Chem. 34 , 2543 (1969)).

Die Herstellung N-geschützter Aminosäurealdehyde erfolgt nach B. Castro et. al. (Synthesis 1983, 676).

Die Aldol-analoge Additon eines N-geschützten Aminosäurealdehydes (bevorzugterweise N-tert.-Butoxycarbonyl-und Benzyloxycarbonyl-Schutzgruppen) erfolgt in einem gegenüber geeigneten Basen inerten Lösungsmittel, wie Ether, THF, Toluol, DMF, DMSO oder Dimethoxyethan.

Als Basen zur Deprotonierung der Arylalkyl-Komponente können Alkalimetallalkoholate, wie Kalium-0-tert.-butylat, Natriummethylat, Alkalimetallhydride, wie Natrium-oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die in den erfindungsgemäßen Verbindungen der allgemeinen Formel I enthaltenen substituierten 4-Amino-3-hydroxybuttersäuren sind literaturbekannt und werden nach D.H. Rich et. al. J. Org. Chem. 43, 3624 (1978) hergestellt. Die zur Herstellung von Verbindungen der Formel I erforderlichen Vor-und Nachoperationen wie Einführung und Ab spaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, in dem man z.B. eine Verbindung der Formel I mit einer basischen Gruppe mit einer stöchiometrischen Menge einer geeigneten Säure umsetzt. Stereoisomerengemische, insbesondere Diastereomerengemische, die bei Verwendung racemischer Aminosäure A oder B anfallen, können in an sich bekannter Weise, beispielsweise durch fraktionierte Kristallisation oder durch Chromatographie getrennt werden. Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen welches als Folge verschiedener Stimuli (Volumendepletion, Natriummangel, β-Rezeptorenstimulation) von den juxtagolmerulären Zellen der Niere in den Blutkreislauf sezerniert wird. Dort spaltet es von dem aus der Leber ausgeschiedenem Angiotensinogen das Decapeptid Angiotensin 1 ab. Dieses wird durch das "angiotensin converting enzyme" (ACE) in Angiotensin 2 überführt. Angiotension 2 spielt eine wesentliche Rolle bei der Blutregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin 1, was eine verminderte Bildung von Angiotension 2 zur Folge hat. Die Erniedrigung der Konzentration dieses aktiven Peptidhormons ist die dirkete Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin 1 in verschiedenen Systemen (Humanplasma, Schweine-Renin) gemessen. Hierzu wird z.B. Humanplasma, welches sowohl Renin als auch Angiotensiongen enthält, bei 37°C mit der zu testenden Verbindung inkubiert. Anschließend wird die Konzentration des während der Inkubation gebildeten Angiotensin 1 mit einem Radioimmunoassay gemessen. Die in der vorliegenden Erfindung beschriebenen Verbindungen der allgemeinen Formel I zeigen in den verwendeten in-vitro-Tests Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam. Die in der vorliegenden Erfindung beschriebenen Verbindungen der allgemeinen Formel I können als Antihypertensiva, sowie zur Behandlung der Herzinsuffizienz verwendet werden.

Die Erfindung betrifft daher auch die Verwendung von Verbindungen der Formel I als Heilmittel und pharmazeutische Präparate, die diese Verbindungen enthalten. Bevorzugt ist die Anwendung beim Menschen.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subcutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkohlische oder ölige Lösungen. Als inerte Träger können z.B. Gummi arabicum, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken-oder Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose-oder Mannitlösungen, oder auch eine Mischung aus den verschiedenen genannten Lösungsmitteln.

Verzeichnis der verwendeten Abkürzungen:

AA Aminosäureanalyse
Ac Acetyl
ACHPA [3S,4S]-4-Amino-3-hydroxy-5-cyclohexyl-pentansäure
BOC tert.Butoxycarbonyl
DC Dünnschichtchromatographie
DCC Dicyclohexylcarbodiimid
DNP 2,4-Dinitrophenyl
DMF Dimethylformamid
DMSO Dimethylsulfoxid
EE Essigsäureethylester
Etoc Ethoxycarbonyl
FAB Fast atom bombardment
HOBt 1-Hydroxybenzotriazol
Iva Isovaleryl
M Molekularpeak
MeOH Methanol
MS Massenspektrum
MTB Methyl-t-butylether
R.T. Raumtemperatur
Schmp. Schmelzpunkt
Sta [3S,4S]-4-Amino-3-hydroxy-6-methyl-heptansäure
Thi β-2-Thienylalanin
THF Tetrahydrofuran
Z Benzyloxycarbonyl

Die sonstigen für Aminosäuren verwendeten Abkürzungen entsprechen dem in der Peptidchemie üblichen drei Buchstaben-Code wie er z.B. in Europ. J. Biochem. 138, 9 - 37 (1984) beschrieben ist. Falls nicht ausdrücklich anders angegeben handelt es sich immer um Aminosäuren der L-Konfiguration.

Die nachstehenden Beispiele dienen zur Erläuterung der vorliegenden Erfindung, ohne daß diese darauf beschränkt wäre.

Beispiel 1

Boc-Phe-His-Sta-N-[(1S,2RS)-2-hydroxy-1-methyl-3-(2-pyridyl)-propyl]-amid:

140 mg Boc-Phe-His(DNP)-Sta-N-[(1S,2RS)-2 hydroxy-1-methyl-3-(2-pyridyl)-propyl]-amid werden in 7 ml DMF mit 0,6 ml Thiophenol 2 h bei R.T. gerührt. Man engt im Vakuum ein und digeriert 3 mal mit Diisopropylether. Nach Chromatographie an Kieselgel (Laufmittel EE/Methanol 3:1) und Einengen der produkthaltigen Fraktion wird die Titelverbindung als farbloses Pulver erhalten.
$R_f$ (EE/Methanol 3:1) = 0,5 MS(FAB) : 708 (M+1).

Das Ausgangsmaterial BOC-Phe-His(DNP)-Sta-N-[(1S,2RS)-2-hydroxy-1-methyl-3-(2-pyridyl)-propyl]-amid wird nach der folgenden Arbeitsvorschrift hergestellt:

a) Zu 193 mg Boc-Phe-His(DNP)-OH, 110 mg H-Sta-N-[(1S,2RS)-2 hydroxy-1-methyl-3-(2-pyridyl)-propyl]-amid und 70 mg HOBt, gelöst in 6 ml THF, werden bei 0 - 5°C 77 mg DCC in 4 ml THF zugetropft. Nach 4 h bei R.T. wird filtriert, eingeengt und in EE gelöst. Man extrahiert 2 mal mit 2 n wäßr. $K_2CO_3$-Lösung, einmal mit Wasser, trocknet über $MgSO_4$ und engt im Vakuum ein. Nach Chromatographie an Kieselgel (Laufmittel EE/Methanol 10:1) erhält man die Titelverbindung als schwach gelbliches Pulver.
$R_f$ (EE/Methanol 10:1) - 0,3 MS(FAB) : 874 (M+1)

b) Herstellung von H-Sta-N-[(1S,2RS)-2-hydroxy-1-methyl-3-(2-pyridyl)-propyl]-amid

211 mg Boc-Sta-N-[(1S,2RS)-2-hydroxy-1-methyl-3-(2-pyridyl)-propyl]-amid werden in 5 ml Dioxan gelöst. Unter Eiskühlung werden 10 ml mit HCl gesättigtes Dioxan zugetropft. Nach 3 h bei R.T. wird im Vakuum eingeengt, in Wasser gelöst, einmal mit $CH_2Cl_2$ extrahiert, mit ges. wäßr. $Na_2CO_3$-Lösung auf pH = 9 gestellt und 3 mal mit $CH_2Cl_2$ extrahiert. Nach Trocknung über $MgSO_4$ und Einengen im Vakuum erhält man die Titelverbindung als farblosen Schaum, der ohne weitere Reinigung in den nächsten Kopplungsschritten weiter eingesetzt werden kann.
MS (FAB) : 324 (M+1)

c) Boc-Phe-His(DNP)-OH wird nach der Aktivestermethode aus Boc-Phe-ONSucc und H-His(DNP)-OH hergestellt (J. Amer. Chem. Soc. 86 1839).

d) Herstellung von Boc-Sta-N-[(1S,2RS)-2-hydroxy-1-methyl-3-(2-pyridyl)-propyl]-amin

Aus 675 mg Boc-Sta-OH (Herstellung nach J. Org. Chem. 43, 3624 (1978)), 400 mg N-[(1S,2RS)-2-hydroxy-1-methyl-3-(2-pyridyl)-propyl]-amin,500 mg HOBt und 550 mg DCC wird analog Vorschrift a) die Titelverbindung als farbloses amorphes Pulver erhalten.
$R_f$ (EE/Methanol 25:1) = 0,3 MS (FAB) : 424 (M+1)

e) Herstellung von N-[(1S,2RS)-2-hydroxy-1-methyl-3-(2-pyridyl)-propyl]-amin:

Analog b), ausgehend von 1,0 g Boc-(2RS,3S)-3-[1-(2-Pyridyl)-2-hydroxy]amin, wird die Titelverbindung als farbloses Öl erhalten.
MS (FAB) : 324 (M+1)

f) Herstellung von Boc-N-[(1S,2RS)-2-hydroxy-1-methyl-3-(2-pyridyl)-propyl]-amin

Zu einer Lösung aus 8,1 g 2-Picolin in 60 ml abs. THF tropft man unter Argon bei -30°C 54 ml einer 1,6 M Lösung von n-Butyllithium in n-Hexan. Nach 30 min bei R.T. wird die tiefrote Lösung auf -10°C gekühlt und 7,5 g Boc-Alaninal (Hergestelltnach Synthesis 1983 676) in 60 ml abs. THF zugetropft. Anschließend wird 15 min bei R.T. gerührt, 100 ml Eiswasser zugegeben und 3 mal mit EE extrahiert. Die organische Phase wird über $MgSO_4$ getrocknet und im Vakuum eingeengt. Nach Chromatographie an Kieselgel erhält man die Titelverbindung als farbloses Öl, Diastereomerenverhältnis 1:2.
$R_f$ (EE) = 0,3 MS (FAB) : 267 (M+1)

Beispiel 2

Boc-Phe-His-ACHPA-N-[1S,2RS)-2-hydroxy-1-methyl-3-(2-pyridyl)-propyl]amid:

150 mg Boc-Phe-His(DNP)-ACHPA-N-[1S,2RS)-2-hydroxy-1-methyl-3-(2-pyridyl)-propyl]-amid werden analog Beispiel 1 mit Thiophenol zur Titelverbindung umgesetzt.
$R_f$ (EE/Methanol 4:1) = 0,3 MS (FAB) : 748 (M+1)

a) Boc-Phe-His(DNP)-ACHPA-N-[1S,2RS)-2-hydroxy-1-methyl-3-(2-pyridyl)-propyl]-amid:

Zu einer Lösung von 390 mg Boc-Phe-His(DNP)-OH, 61 µl Pyridin und 100 µl N-Ethylpiperidin in 15 ml $CH_2Cl_2$ wird bei -10°C 93 µl Pivaloylchlorid in 3 ml $CH_2Cl_2$ zugetropft. 20 min wird bei +5 bis 10°C gerührt, auf -15°C abgekühlt und 250 mg H-ACHPA-N-[(1S,2RS)-2-hydroxy-1-methyl-3-(2-pyridyl)-propyl]-amid in 5 ml $CH_2Cl_2$ zugetropft. 3 h wird bei R.T. gerührt, im Vakuum eingeengt und in 120 ml EE aufgenommen. Man extrahiert 3 mal mit 2 n wäßr. $K_2CO_3$-Lösung, einmal mit Wasser, trocknet über $Na_2SO_4$ und engt im

Vakuum ein. Nach Chromatographie an Kieselgel (Laufmittel EE/Methanol 7:1) erhält man die Titelverbindung als schwach gelbliches Pulver.
$R_f$ (EE/Methanol 7:1) = 0,4 MS (FAB) : 914 (M+1)

b) H-ACHPA-N-[(1S,2RS)-2-hydroxy-1-methyl-3-(2-pyridyl)-propyl]-amid:

450 mg Boc-ACHPA-N-[(1S,2RS)-2-hydroxy-1-methyl-3-(2-pyridyl)-propyl]-amid werden analog Beispiel 1b), mit Dioxan/HCl zur Titelverbindung umgesetzt.
MS (FAB) : 364 (M+1)

c)Boc-ACHPA-(2RS,3S)-3-[1-(2-Pyridyl)-2-hydroxybutyl]amid:

Analog Beispiel 1a) werden 640 mg Boc-ACHPA-Oh [Herstellung nach J. Med. Chem. 28, 1779 (1985)] und 500 mg N-[(1S,2RS)-2-hydroxy-1-methyl-3-(2-pyridyl)-propyl]-amin mit DCC/HOBt gekoppelt. Nach Chromatographie an Kieselgel (Laufmittel EE/Methanol 10:1) erhält man die Titelverbindung als farbloses Pulver.
$R_f$ (EE/Methanol 10:1) = 0,5 MS (FAB) : 464 (M+1)

Die Verbindungen der Beispiele 3 - 8 werden analog Beispiel 2 ausgehend von H-ACHPA-N-[(1S,2RS)-2-hydroxy-1-methyl-3-(2-pyridyl)-propyl]-amid und dem entsprechenden Dipeptidderivat hergestellt.

| Beispiel Nr. | Verbindung |
|---|---|
| 3 | Z-Phe-His-ACHPA-N-[(1S,2RS)-2-hydroxy-1-methyl-3-(2-pyridyl)-propyl]-amid |
| 4 | Iva-Phe-His-ACHPA-N-[(1S,2RS)-2-hydroxy-1-methyl-3-(2-pyridyl)-propyl]-amid |
| 5 | Etoc-Phe-His-ACHPA-N-[(1S,2RS)-2-hydroxy-1-methyl-3-(2-pyridyl)-propyl]-amid |
| 6 | Boc-Thi-His-ACHPA-N-[(1S,2RS)-2-hydroxy-1-methyl-3-(2-pyridyl)-propyl]-amid |
| 7 | Etoc-Thi-His-ACHPA-N-[(1S,2RS)-2-hydroxy-1-methyl-3-(2-pyridyl)-propyl]-amid |
| | Iva-Thi-His-ACHPA-N-[(1S,2RS)-2-hydroxy-1-methyl-3-(2-pyridyl)-propyl]-amid |

Beispiel 9

(2S)-Hydroxy-3-phenyl-propionyl-His-ACHPA-N-[(1S-2RS)-2-hydroxy-1-methyl-3-(2-pyridyl)-propyl]-amid:

130 mg Hydroxy-3-phenyl-propionyl-His(DNP)-ACHPA-N-[(1S-2RS)-2-hydroxy-1-methyl-3-(2-pyridyl)-propyl]-amid werden analog Beispiel 1 mit Thiophenol zur Titelverbindung umgesetzt.
$R_f$ (EE/Methanol/Methyl-t-butylether 1:1:1) = 0,3 MS (FAB) : 649 (M+1)

a) (2S)-Hydroxy-3-phenyl-propinyl-His(DNP)-ACHPA-N-[(1S-2RS)-2-hydroxy-1-methyl-3-(2-pyridyl)-propyl]-amid:

83 mg Hydroxy-3-phenyl-propionsäure [Herstellung nach J. Am. Chem. Soc. 86, 5329 (1964)] und 300 mg H-His-(DNP)-ACHPA-N-[(1S,2RS)-2-hydroxy-1-methyl-3-(2-pyridyl)-propyl]-amid werden analog Beispiel 1a) mit DCC/HOBt zur Titelverbindung umgesetzt.
$R_f$ (EE/Methanol 8:1) = 0,3 MS (FAB) : 815 (M+1)

b) H-His-(DNP)-ACHPA-N-[(1S-2RS)-2-hydroxy-1-methyl-3-(2-pyridyl)-propyl]-amid:

550 mg Boc-His(DNP)-ACHPA-N-[(1S-2RS)-2-hydroxy-1-methyl-3-(2-pyridyl)-propyl]-amid werden analog Beispiel 1b) mit Dioxan/HCl zur Titelverbindung umgesetzt, die ohne Reinigung weiter umgesetzt wird.
MS (FAB) : 667 (M+1)

c) Boc-His(DNP)-ACHPA-N-[(1S-2RS)-2-hydroxy-1-methyl-3-(2-pyridyl)-propyl]-amid:

Analog Beispiel 2a) werden 290 mg Boc-His(DNP)-OH und 250 mg H-ACHPA-N-[(1S-2RS)-2-hydroxy-1-methyl-3-(2-pyridyl)-propyl]-amid mit Pivaloylchlorid gekoppelt. Nach Chromatographie an Kieselgel (EE/Methanol 4:1) erhält man die Titelverbindung als schwach gelbliches Pulver.
$R_f$ (EE/Methanol 4:1) 0,4 MS (FAB) : 767 (M+1)

Beispiel 10

(2S)-Hydroxy-3-phenylpropionyl-His-ACHPA-N-[(1S)-1-methyl-3-(2-pyridyl)-propyl]-amid:

232 mg (2S)-Hydroxy-3-phenyl-propionyl-His(DNP)-ACHPA-N-[(1S)-1-methyl-3-(2-pyridyl)-propyl]-amid werden analog Beispiel 1 mit Thiophenol zur Titelverbindung umgesetzt. $R_f$ (EE/Methanol/Methyl-t-Butylether 1:1:1) = 0,4
MS (FAB) : 633 (M+1)

a) (2S)-Hydroxy-3-phenyl-propionyl-His(DNP)-ACHPA-N-[(1S)-1-methyl-3-(2-pyridyl)-propyl]-amid:

167 mg H-His(DNP)-ACHPA-N-[(1S)-1-methyl-3-(2-pyridyl)-propyl]-amid und 61 mg (2S)-Hydroxy-3-phenyl-propionsäure werden analog Beispiel 1a) mit DCC/HOBt zur Titelverbindung umgesetzt.
$R_f$ (EE/Methanol 8:1) = 0,4 MS (FAB) : 799 (M+1)

b) H-His(DNP)-ACHPA-N-[(1S)-1-methyl-3-(2-pyridyl)-propyl]-amid:

202 mg Boc-His(DNP)-ACHPA-N-[(1S)-1-methyl-3-(2-pyridyl)-propyl]-amid werden analog Beispiel 1b) mit Dioxan/HCl zur Titelverbindung umgesetzt, die ohne Reinigung weiter umgesetzt wird.
MS (FAB) : 651 (M+1)

c) Boc-His(DNP)-ACHPA-N-[(1S)-1-methyl-3-(2-pyridyl)-propyl]-amid:

660 mg H-ACHPA-N-[(1S)-1-methyl-3-(2-pyridyl)-propyl]-amid und 800 mg Boc-His(DNP)-OH werden analog Beispiel 2a) mit Pivaloychlorid gekoppelt. Nach Chromatographie an Kieselgel (EE/Methanol 4:1) erhält man die Titelverbindung als schwach gelbliches Pulver.
$R_f$ (EE/Methanol 4:1) = 0,5 MS (FAB) 751 (M + 1)

d) H-ACHPA-N-[(1S)-1-methyl-3-(2-pyridyl)-propyl]-amid:

940 mg Boc-ACHPA-N-[(1S)-1-methyl-3-(2-pyridyl)-propyl]-amid werden analog Beispiel 1b) mit Dioxan/HCl zur Titelverbindung umgesetzt, die ohne Reinigung weiter umgesetzt wird.
MS (FAB) : 651 (M + 1)

e) Boc-ACHPA-N-[(1S)-1-methyl-3-(2-pyridyl)-propyl]-amid:

Analog Beispiel 1a) werden 2,0 g Boc-ACHPA-OH und 0,95 g N-[(1S)-1-Methyl-3-(2-pyridyl)-propyl]-amin mit DCC/HOBt gekoppelt. Nach Chromatographie an Kieselgel (Laufmittel EE) erhält man die Titelverbindung als farbloses Pulver.
$R_f$ (EE) = 0,1 MS (FAB) : 448 (M + 1)

f) N-[(1S)-1-Methyl-3-(2-pyridyl)-propyl]-amin:

17,1 g N-[(1S)-1-Methyl-3-(2-pyridyl)-propyl]-amin werden in 100 ml Dioxan gelöst und 200 ml mit HCl gesättigtes Dioxan zugetropft. Nach 2 h bei R.T. wird im Vakuum eingeengt, in Wasser gelöst, einmal mit $CH_2Cl_2$ extrahiert, mit ges. wäßr. $Na_2CO_3$-Lösung auf pH = 9 gestellt und 3 x mit $CH_2Cl_2$ extrahiert. Nach Trocknung über $Na_2SO_4$ und Einengen im Vakuum erhält man die Titelverbindung als farblosen Schaum, der ohne Reinigung weiter eingesetzt werden kann.
$R_f$ (Aceton/Methanol 2:1, 1 % Triethylamin) = 0,2 MS : 151 (M + 1)
Zur Aufbewahrung kann das Monohydrochlorid hergestellt und aus Isopropanol umkristallisiert werden.
Schmp. 187°C

g) Boc-N-[(1S)-1-Methyl-3-(2-pyridyl)-propyl]-amin:

19,2 g Boc-N-[(1S)-1-methyl-3-(2-pyridyl)-2-propenyl]-amin werden mit 1,9 g Pd/C in 100 ml Ethanol bei Normaldruck hydriert. Man erhält die Titelverbindung als farblosen Feststoff.
$R_f$ (EE/Diisopropylether 2:1) = 0,1
Schmp. 82°C

h) Boc-N-[(1S)-1-methyl-3-(2-pyridyl)-2-propenyl]-amid:

37,6 g 2-Picolyl-triphenylphosphoniumchlorid und 10,3 g Kalium-tert.-butylat werden 3 h bei R.T. in abs. THF unter Argon gerührt. Bei R.T. wird eine Lösung von 15,2 g Boc-Alaninal (hergestellt nach Synthesis 1983, 676) in abs. THF zugetropft. Nach 30 min bei R.T. wird im Vakuum eingeengt und Chromatographie an Kieselgel (Laufmittel Diisopropylether) liefert die Titelverbindung als farblosen Feststoff.
$R_f$ (Diisopropylether) = 0,2 MS : 249 (M + 1)
Schmp.: 92 - 94°C

Beispiel 11

(2S)-Hydroxy-3-(2-thienyl)-propionyl]-His-ACHPA-N-[(1S)-1-methyl-3-(2-pyridyl)-propyl]-amid

Die Titelverbindung wird analog Beispiel 9 hergestellt, Chromatographie an Kieselgel (Laufmittel EE/Methanol/Methyl-t-Butylether 1:1:1) liefert ein farbloses Pulver.

$R_f$ (EE/Methanol/Methyl-t-Butylether 1:1:1) = 0,4

MS (FAB : 639 (M+1)

Die analytischen Daten der Verbindungen der Beispiele 1 - 10 sind in Tabelle 1 zusammengefaßt.

Tabelle 1:

| Bsp. Nr. | MS (FAB) | DC ($R_f$) | | $^1$H - NMR a) |
|---|---|---|---|---|
| 1 | 708 (M+1) | 0,5 | (EE/MeOH 3:1) | + |
| 2 | 748 (M+1) | 0,3 | (EE/MeOH 4:1) | + |
| 3 | 782 (M+1) | 0,3 | (EE/MeOH 4:1) | + |
| 4 | 732 (M+1) | 0,3 | (EE/MeOH 4:1) | + |
| 5 | 720 (M+1) | 0,4 | (EE/MeOH 3:1) | + |
| 6 | 754 (M+1) | 0,3 | (EE/MeOH 4:1) | + |
| 7 | 726 (M+1) | 0,4 | (EE/MeOH 3:1) | + |
| 8 | 738 (M+1) | 0,3 | (EE/MeOH 4:1) | + |
| 9 | 649 (M+1) | 0,3 | (EE/MeOH/MTB 1:1:1) | + |
| 10 | 633 (M+1) | 0,4 | (EE/MeOH/MTB 1:1:1) | + |
| 11 | 639 (M+1) | 0,4 | (EE/MeOH/MTB 1:1:1) | + |

a) gemessen bei 270 MHz, + bedeutet: in Einklang mit der angegebenen Struktur

**Ansprüche**

1. Verbindung der Formel I

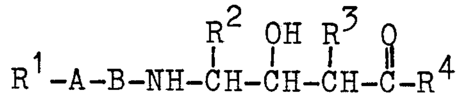

(I)

in welcher
$R^4$ einen Rest der Formel II bedeutet,

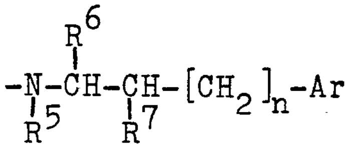

worin $R^5$ für Wasserstoff oder $(C_1-C_7)$-Alkyl und $R^6$ für Wasserstoff, $(C_1-C_7)$-Alkyl oder durch Hydroxy, $(C_1-C_5)$-Alkoxy, $(C_1-C_5)$Alkyl-thio, Carboxy, $(C_1-C_5)$-Alkoxycarbonyl, Cl, Br, $(C_1-C_5)$-Alkylamino, Di-$(C_1-C_5)$-alkylamino, $(C_1-C_5)$-Alkoxycarbonylamino oder $(C_1-C_{15})$-Aralkyloxycarbonylamino monosubstituiertes $(C_1-C_7)$-Alkyl stehen,
$R^7$ $a_1$) falls A wie unten unter ($c_2$) definiert ist, OH bedeutet,
$a_2$) andernfalls wie $R^6$ definiert ist oder OH bedeutet,
n 0, 1, 2, 3 oder 4 ist und
Ar für $(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-Alkylamino, Carboxy, Carboxymethyloxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo und Guanidinomethyl substituiert ist, oder für den Rest eines 5-oder 6-gliedrigen monocyclischen oder 9-oder 10-gliedrigen bicyclischen Heteroaromaten mit mindestens 1 C-Atom, 1 - 3 N-Atomen und/oder 1 S-oder 0-Atom als Ringglieder steht, wobei Ar auch über -0-gebunden sein kann,
$R^1$ $b_1$) abwesend ist oder Wasserstoff bedeutet,
$b_2$) $(C_1-C_{20})$-Alkyl, das gegebenenfalls durch ein, zwei oder drei gleiche oder verschiedene Reste aus der Reihe Hydroxy, $(C_1-C_7)$-Alkoxy, $(C_1-C_7)$-Alkanoyloxy, Carboxy, $(C_1-C_7)$-Alkoxycarbonyl, $(C_1-C_8)$-Alkanoyloxy, Cl, Br, Amino, $(C_1-C_7)$-Alkylamino, Di-$(C_1-C_7)$-alkylamino, $(C_1-C_5)$-Alkoxycarbonylamino oder $(C_7-C_{11})$-Aralkyloxycarbonylamino substituiert ist,
$(C_3-C_8)$-Cycloalkyl,
$(C_3-C_8)$-Cycloalkyl-$(C_1-C_{10})$alkyl oder
$(C_6-C_{14})$-Aryl-$(C_1-C_8)$-alkyl, das im Arylteil gegebenenfalls durch einem oder zwei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist, bedeutet oder
$b_3$) einen Rest der Formel III bedeutet,
$R^a$-W-(III)
worin W für -CO-, -O-CO-, $-SO_2$-oder -NH-CO-steht und
$R^a$ Wasserstoff, $(C_1-C_{10})$-Alkyl, das gegebenenfalls ein-oder zweifach ungesättigt ist und das gegebenenfalls durch bis zu 3 gleiche oder verschiedene Rest aus der Reihe Hydroxy, $(C_1-C_7)$-Alkoxy$(C_1-C_7)$-Alkanoyloxy, Carboxy, $(C_1-C_7)$-Alkoxycarbonyl, Cl, Br, Amino, $(C_1-C_7)$-Alkylamino, Di-$(C_1-C_7)$-Alkylamino, $(C_1-C_5)$-Alkoxycarbonylamino, $(C_7-C_{15})$-Aralkoxycarbonylamino oder 9-Fluorenylmethoxycarbonylamino monosubstituiert ist,
$(C_3-C_8)$-Cycloalkyl,
$(C_3-C_8)$-Cycloalkyl-$(C_1-C_6)$alkyl, $(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino gegebenenfalls mit bis zu 2 Halogen substituiertes Anilino und Trifluormethyl substituiert ist,
$(C_6-C_{14})$-Aryl-$(C_1-C_6)$-alkyl, worin der Arylteil gegebenenfalls wie vorstehend bei Aryl definiert ist,
Heteroaryl oder Heteroaryl-$(C_1-C_6)$-alkyl, wobei der Rest des Heteroaromaten jeweils wie Ar bei $R^4$ definiert ist, bedeutet,
A $c_1$) einen Rest der Formeln IV bedeutet,

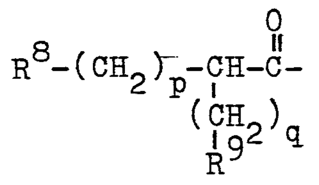

(IV)

in welcher

p und q gleich oder verschieden sind und 0, 1, 2, 3 oder 4 bedeuten,

$R^8$ und $R^9$ gleich oder verschieden sind und Phenyl, 2-oder 3-Thienyl, 2-, 3-oder 4-Pyridyl, 1-, 2-oder 4-Imidazolyl, OH oder Wasserstoff bedeuten oder

$c_2$) einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, 0-Methyltyrosin, 0-Benzyltyrosin, 0-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Norleucin , 1,2,3,4-Tetrahydroisochinolin-3-carbonsäure, Homophenylalanin, DOPA, 0-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin bedeutet,

B einen Rest einer Aminosäure, wie unter ($c_2$) definiert, bedeutet,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet und

$R^3$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet,

sowie deren physiologisch verträglichen Salze.

2. Verbindung der Formel I gemäß Anspruch 1, in welcher $R^5$ Wasserstoff oder Methyl bedeutet und/oder in welcher $R^6$ Wasserstoff, Methyl, Ethyl, Propyl, Isopropyl, Butyl, Isobutyl, sec. Butyl, ($C_1$-$C_5$)-Alkoxycarbonylmethyl), ($C_1$-$C_5$)-Alkoxycarbonylethyl oder Methylthioethyl bedeutet und/oder in welcher $R^7$

$a_1$) falls A wie im Anspruch 1 unter ($c_2$) definiert ist, OH bedeutet,

$a_2$) andernfalls Wasserstoff oder OH bedeutet.

3. Verbindung der Formel I gemäß Anspruch 1 oder 2, in welcher Ar für Phenoxy, Phenyl, 2-, 3-oder 4-Pyridyl, 4-Imidazolyl, 1-, 3-oder 4-Isochinolyl, 2-, 3-oder 4-Carboxyphenyl, 2-, 3-oder 4-($C_1$-$C_7$)-Alkoxycarbonylphenyl, vorzugsweise 3-Methoxycarbonylphenyl oder 3-tert.-Butoxycabonylphenyl, 2-, 3-oder 4-Aminophenyl, Mono-oder Di-($C_1$-$C_7$)-alkylaminophenyl, vorzugsweise 3-Methylaminophenyl oder 3-Dimethylaminophenyl, 2-, 3-oder 4-Carbamoylphenyl, 3-Sulfamoylphenyl, 3-Sulfophenyl, Amino-($C_1$-$C_7$)-alkylphenyl, vorzugsweise 2-, 3-oder 4-Aminomethylphenyl, 3-(Methylaminomethyl)-phenyl, 3-(N,N-Dimethylaminomethyl)-phenyl, ($C_1$-$C_7$)-Alkoxycarbonylmethoxyphenyl, vorzugsweise 2-, 3-oder 4-Methoxycarbonylmethoxyphenyl oder 3-tert.-Butoxycarbonylmethoxyphenyl, 2-, 3-oder 4-Carboxymethyloxyphenyl, ($C_1$-$C_7$)-Alkoxyphenyl, vorzugsweise 2-, 3-oder 4-Methoxyphenyl, oder 2-, 3-oder 4-Guanidinomethylphenyl steht und/oder in welcher $R^1$ a) ($C_1$-$C_{11}$)-Alkanoyl, vorzugsweise n-Decanoyl, Formyl, Acetyl, Pivaloyl, Isovaleryl oder Isobutyryl, gegebenenfalls geschütztes Amino-($C_1$-$C_{11}$)-alkanoyl, vorzugsweise 4-Aminobutyryl, 5-Aminopentanoyl, 6-Aminohexanoyl, 4-N-tert.-Butoxycarbonylaminobutyryl, 5-N-tert.Butoxycarbonylaminopentanoyl oder 6-N-tert.-butoxycarbonylaminohexanoyl, Di-($C_1$-$C_7$)-alkylamino-($C_1$-$C_{11}$)-alkanoyl, vorzugsweise Dimethylaminoacetyl, ($C_4$-$C_9$)-Cycloalkanoyl, vorzugsweise Cyclopropanoyl, Cyclobutanoyl, Cyclopentanoyl oder Cyclohexanoyl, ($C_6$-$C_{10}$)-Aryl-($C_1$-$C_{11}$)-alkanoyl, vorzugsweise Phenylacetyl, Phenylpropanoyl oder Phenylbutanoyl, 2-(o,o-Dichloroanilino)-phenylacetyl, 2-(N-Benzyl-o,o-dichloranilino)-phenylacetyl, gegebenenfalls durch Halogen, ($C_1$-$C_7$)-Alkyl, ($C_1$-$C_7$)-Alkoxy, ($C_1$-$C_7$)-Alkoxycarbonyl substituiertes Benzoyl, vorzugsweise 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzylsulfonyl, ($C_1$-$C_{10}$)-Alkoxycarbonyl, vorzugsweise Methoxycarbonyl oder tert.-Butoxy-carbonyl, durch Halogen substituiertes ($C_1$-$C_{10}$)-Akoxycarbonyl, vorzugsweise 2,2,2-Trichlorethoxycarbonyl oder 1,1-Dimethyl-2,2,2-trichlorethoxycarbonyl, ($C_6$-$C_{14}$)-Aryl-($C_1$-$C_6$)-alkoxycarbonyl, vorzugsweise Benzyloxycarbonyl oder 9-Fluorenylmethylcarbonyl, (2S)-trans-4-Hydroxy-1-acetylpyrrolidinyl-2-carbonyl, $\alpha$-Methoxycarbonyl-$\alpha$-benzyloxycarbonylaminoacetyl, N-Acetyl-(2S,3aS,6aS)-octahydrocyclopenta[b]pyrrol-2-carbonyl oder N-Acetyl-(2R,3aR,6aR)-octahydrocyclopenta[b]pyrrol-2-carbonyl bedeutet, oder in welcher $R^1$ b) abwesend ist, Wasserstoff bedeutet oder für ($C_1$-$C_{10}$)-Alkyl, Cyclopentyl, Cyclohexyl, Cyclopentyl-($C_1$-$C_{10}$)-alkyl, Cyclohexyl-($C_1$-$C_{10}$)-alkyl, gegebenenfalls substituiertes Phenyl-($C_1$-$C_8$)-alky, $H_2N$-($C_1$-$C_{10}$)-Alkyl, HO-($C_1$-$C_{10}$)-Alkyl, ($C_1$-$C_4$)-Alkoxy-($C_1$-$C_{10}$)-alkyl, ($C_1$-$C_4$)-Alkoxycarbonyl-($C_1$-$C_{10}$)-alkyl, Carboxy-($C_1$-$C_{10}$)-alkyl oder ($C_1$-$C_8$)-Alkanoyloxy-($C_1$-$C_{10}$)-alkyl steht. .

4. Verbindung der Formel I gemäß einem der Ansprüche 1 bis 3, in welcher $R^4$ einen Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, 4-Chlorphenylalanin, Methioninsulfon, Methioninsulfoxid, 3-Pyridylalanin, 3-Pyridylalanin, Cyclohexylalaninm Cyclohexylglycin, im-Methylhistidin, 0-Methyltyrosin, 0-Benzyltyrosin, 0-tert.-Butyltyrosin, Phenylglycin, 1-Naphthylalanin, 2-Naphtylalanin, 4-Nitrophenylalanin, Norleucin, 1,2,3,4-Tetrahydroisochinolin-3-carbonsäure, Homophenylalanin, 2-Amino-4-(2-thienyl)-buttersäure, (Z)-Dehydrophenylalanin oder (E)-Dehydrophenylalanin bedeutet.

5. Verbindung der Formel I gemäß einem der Ansprüche 1 bis 4, in welcher B einen N-terminal mit A und C-terminal mit der NH-Gruppe von Formel I verknüpften Rest einer Aminosäure bedeutet, die wie die Aminosäuren in Anspruch 4 definiert ist.

6. Verbindung der Formel I gemäß einem der Ansprüche 1 bis 5, in welcher A einen N-terminal mit $R^1$ und C-terminal mit B verknüpften Rest einer Aminosäure bedeutet, die wie die Aminosäuren im Anspruch 4 definiert ist, (2S)-O-Benzyl-3-phenyl-propanoyl oder (2R,S)-2-Benzyl-4-oxo-6,6-dimethyl-6-heptanoyl bedeutet.

7. Verbindung der Formel I gemäß einem der Ansprüche 1 bis 6, in welcher $R^2$ Cyclohexylmethyl, Benzyl oder Isobutyl bedeutet.

8. Verbindung der Formel I gemäß einem der Ansprüche 1 bis 7, in welcher $R^3$ Wasserstoff, Ethyl, sec.-Butyl, Isobutyl, Benzyl, Phenethyl, oder 3-Phenylpropyl bedeutet.

9. Verfahren zur Herstellung einer Verbindung der Formel I gemäß einem der Ansprüche 1 bis 8, dadurch gekennzeichnet, daß man ein Fragment mit endständiger Carboxylgruppe oder reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

10. Verbindung gemäß einem der Ansprüche 1 bis 8 zur Anwendung als Heilmittel.

11. Verwendung einer Verbindung gemäß einem der Ansprüche 1 bis 8 und 10 zur Anwendung als Heilmittel bei der Behandlung des Bluthochdrucks.

12. Pharmazeutisches Mittel enthaltend eine Verbindung gemäß einem der Ansprüche 1 bis 8, 10 und 11.

Patentansprüche für die folgenden Vertragsstaaten: Griechenland, Österreich und Spanien:

1. Verfahren zur Herstellung einer Verbindung der Formel I

$$R^1-A-B-NH-\overset{R^2}{\overset{|}{C}}H-\overset{OH}{\overset{|}{C}}H-\overset{R^3}{\overset{|}{C}}H-\overset{O}{\overset{\|}{C}}-R^4 \qquad (I)$$

in welcher

$R^4$ einen Rest der Formel II bedeutet,

$$-\underset{R^5}{\underset{|}{N}}-\overset{R^6}{\overset{|}{C}}H-\underset{R^7}{\underset{|}{C}}H-[CH_2]_n-Ar$$

worin $R^5$ für Wasserstoff oder $(C_1-C_7)$-Alkyl und

$R^6$ für Wasserstoff, $(C_1-C_7)$-Alkyl oder durch Hydroxy, $(C_1-C_5)$-Alkoxy, $(C_1-C_5)$-Alkyl-thio, Carboxy, $(C_1-C_5)$-Alkoxycarbonyl, Cl, Br, $(C_1-C_5)$-Alkylamino, Di-$(C_1-C_5)$-alkylamino, $(C_1-C_5)$-Alkoxycarbonylamino oder $(C_1-C_{15})$-Aralkyloxycarbonylamino monosubstituiertes $(C_1-C_7)$-Alkyl stehen,

$R^7$ $a_1$) falls A wie unten unter ($c_2$) definiert ist, OH bedeutet,

$a_2$) andernfalls wie $R^6$ definiert ist oder OH bedeutet,

n 0, 1, 2, 3 oder 4 ist und

Ar für $(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-Alkylamino, Carboxy, Carboxymethyloxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo und Guanidinomethyl substituiert ist, oder für den Rest eines 5-oder 6-gliedrigen monocyclischen

oder 9-oder 10-gliedrigen bicyclischen Heteroaromaten mit mindestens 1 C-Atom, 1 - 3 N-Atomen und/oder 1 S-oder O-Atom als Ringglieder steht, wobei Ar auch über -O-gebunden sein kann,

$R^1$ $b_1$) abwesend ist oder Wasserstoff bedeutet,

$b_2$) ($C_1$-$C_{20}$)-Alkyl, das gegebenenfalls durch ein, zwei oder drei gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, ($C_1$-$C_8$)-Alkanoyloxy, Cl, Br, Amino, ($C_1$-$C_7$)-Alkylamino, Di-($C_1$-$C_7$)-alkylamino, ($C_1$-$C_5$)-Alkoxycarbonylamino oder ($C_7$-$C_{11}$)-Aralkyloxycarbonylamino substituiert ist,

($C_3$-$C_8$)-Cycloalkyl,

($C_3$-$C_8$)-Cycloalkyl-($C_1$-$C_{10}$)alkyl oder

($C_6$-$C_{14}$)-Aryl-($C_1$-$C_8$)-alkyl, das im Arylteil gegebenenfalls durch einen oder zwei gleiche oder verschiedene Reste aus der Reihe, F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist, bedeutet oder

$b_3$) einen Rest der Formel III bedeutet,

$R^a$-W-(III)

worin W für -CO-, -O-CO-, -$SO_2$-oder -NH-CO-steht und

$R^a$ Wasserstoff, ($C_1$-$C_{10}$)-Alkyl, das gegebenenfalls ein-oder zweifach ungesättigt ist und das gegebenenfalls durch bis zu 3 gleiche oder verschiedene Rest aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-Alkylamino, ($C_1$-$C_5$)-Alkoxycarbonylamino, ($C_7$-$C_{15}$)-Aralkoxycarbonylamino oder 9-Fluorenylmethyloxycarbonylamino monosubstituiert ist,

($C_3$-$C_8$)-Cycloalkyl,

($C_3$-$C_8$)-Cycloalkyl-($C_1$-$C_6$)alkyl, ($C_6$-$C_{14}$)-Aryl, das gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogen substituiertes Anilino und Trifluormethyl substituiert ist,

($C_6$-$C_{14}$)-Aryl-($C_1$-$C_6$)-alkyl, worin der Arylteil gebenenfalls wie vorstehend bei Aryl definiert ist,

Heteroaryl oder Heteroaryl-($C_1$-$C_6$)-alkyl, wobei der Rest des Heteroaromaten jeweils wie Ar bei $R^4$ definiert ist, bedeutet,

A $c_1$) einen Rest der Formeln IV bedeutet,

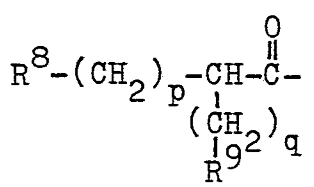

(IV)

in welcher

p und q gleich oder verschieden sind und 0, 1, 2, 3 oder 4 bedeuten,

$R^8$ und $R^9$ gleich oder verschieden sind und Phenyl, 2-oder 3-Thienyl, 2-, 3-oder 4-Pyridyl, 1-, 2-oder 4-Imidazolyl, OH oder Wasserstoff bedeuten oder

$c_2$) einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Norleucin, 1,2,3,4-Tetrahydroisochinolin-3-carbonsäure, Homophenylalanin, DOPA, O-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin bedeutet,

B einen Rest einer Aminosäure, wie unter ($c_2$) definiert, bedeutet,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet und

$R^3$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet,

oder deren physiologisch verträglichen Salzes, dadurch gekennzeichnet, daß man ein Fragment mit endständiger Carboxylgruppe oder reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren gemäß Anspruch 1, dadurch gekennzeichnet, daß eine Verbindung der Formel I hergestellt wird, in welcher $R^5$ Wasserstoff oder Methyl bedeuten und/oder in welcher $R^6$ Wasserstoff, Methyl, Ethyl, Propyl, Isopropyl, Butyl, Isobutyl, sec. Butyl, ($C_1$-$C_5$)-Alkoxycarbonylmethyl, ($C_1$-$C_5$)-Alkoxycarbonylethyl oder Methylthioethyl bedeuten und/oder in welcher $R^7$

$a_1$) falls A wie im Anspruch 1 unter ($C_2$) definiert ist, OH bedeutet,

$a_2$) andernfalls Wasserstoff oder OH bedeutet.

3. Verfahren gemäß Anspruch 1 oder 2, dadurch gekennzeichnet, daß eine Verbindung der Formel I hergestellt wird, in welcher Ar für Phenoxy, Phenyl, 2-, 3-oder 4-Pyridyl, 4-Imidazolyl, 1-, 3-oder 4-Isochinolyl, 2-, 3-oder 4-Carboxyphenyl, 2-, 3-oder 4-($C_1$-$C_7$)-Alkoxycarbonylphenyl, vorzugsweise 3-Methoxycarbonylphenyl oder 3-tert.-Butoxycarbonylphenyl, 2-, 3-oder 4-Aminophenyl, Mono-oder Di-($C_1$-$C_7$)-alkylaminophenyl, vorzugsweise 3-Methylaminophenyl oder 3-Dimethylaminophenyl, 2-, 3-oder 4-Carbamoylphenyl, 3-Sulfamoylphenyl, 3-Sulfophenyl, Amino-($C_1$-$C_7$)-alkylphenyl, vorzugsweise 2-, 3-oder 4-Aminomethylphenyl, 3-(Methylaminomethyl)-phenyl, 3-(N,N-Dimethylaminomethyl)-phenyl, ($C_1$-$C_7$)-Alkoxycarbonylmethoxyphenyl, vorzugsweise 2-, 3-oder 4-Methoxycarbonylmethoxyphenyl oder 3-tert.-Butoxycarbonylmethoxyphenyl, 2-, 3-oder 4-Carboxymethyloxyphenyl, ($C_1$-$C_7$)-Alkoxyphenyl, vorzugsweise 2-, 3-oder 4-Methoxyphenyl, oder 2-, 3-oder 4-Guanidinomethylphenyl steht und/oder

in welcher $R^1$ a) ($C_1$-$C_{11}$)-Alkanoyl, vorzugsweise n-Decanoyl, Formyl, Acetyl, Pivaloyl, Isovaleryl oder Isobutyryl, gegebenenfalls geschütztes Amino-($C_1$-$C_{11}$)-alkanoyl, vorzugsweise 4-Aminobutyryl, 5-Aminopentanoyl, 6-Aminohexanoyl, 4-N-tert.-Butoxycarbonylaminobutyryl, 5-N-tert.Butoxycarbonylaminopentanoyl oder 6-N-tert.-butoxycarbonylaminohexanoyl, Di-($C_1$-$C_7$)-alkylamino-($C_1$-$C_{11}$)-alkanoyl, vorzugsweise Dimethylaminoacetyl, ($C_4$-$C_9$)-Cycloalkanoyl, vorzugsweise Cyclopropanoyl, Cyclobutanoyl, Cyclopentanoyl oder Cyclohexanoyl, ($C_6$-$C_{10}$)-Aryl-($C_1$-$C_{11}$)-alkanoyl, vorzugsweise Phenylacetyl, Phenylpropanoyl oder Phenylbutanoyl, 2-(o,o-Dichloroanilino)-phenylacetyl, 2-(N-Benzyl-o,o-dichloranilino)-phenylacetyl, gegebenenfalls durch Halogen, ($C_1$-$C_7$)-Alkyl, ($C_1$-$C_7$)-Alkoxy, ($C_1$-$C_7$)-Alkoxycarbonyl substituiertes Benzoyl, vorzugsweise 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzylsulfonyl, ($C_1$-$C_{10}$)-Alkoxycarbonyl, vorzugsweise Methoxycarbonyl oder tert.-Butoxy-carbonyl, durch Halogen substituiertes ($C_1$-$C_{10}$)-Alkoxycarbonyl, vorzugsweise 2,2,2-Trichlorethoxycarbonyl oder 1,1-Dimethyl-2,2,2-trichlorethoxycarbonyl, ($C_6$-$C_{14}$)-Aryl-($C_1$-$C_6$)-alkoxycarbonyl, vorzugsweise Benzyloxycarbonyl oder 9-Fluorenylmethylcarbonyl, (2S)-trans-4-Hydroxy-1-acetylpyrrolidinyl-2-carbonyl, α-Methoxycarbonyl-α-benzyloxycarbonylaminoacetyl, N-Acetyl-(2S,3aS,6aS)-octahydrocyclopenta[b]pyrrol-2-carbonyl oder N-Acetyl-(2R,3aR,6aR)-octahydrocyclopenta[b]pyrrol-2-carbonyl bedeutet oder in welcher $R^1$ b) abwesend ist, Wasserstoff bedeutet oder für ($C_1$-$C_{10}$)-Alkyl, Cyclopentyl, Cyclohexyl, Cyclopentyl-($C_1$-$C_{10}$)-alkyl, Cyclohexyl-($C_1$-$C_{10}$)-alkyl, gegebenenfalls substituiertes Phenyl-($C_1$-$C_8$)-alkyl, $H_2N$-($C_1$-$C_{10}$)-Alkyl, HO-($C_1$-$C_{10}$)-Alkyl, ($C_1$-$C_4$)-Alkoxy-($C_1$-$C_{10}$)-alkyl, ($C_1$-$C_4$)-Alkoxycarbonyl-($C_1$-$C_{10}$)-alkyl, Carboxy-($C_1$-$C_{10}$)-alkyl oder ($C_1$-$C_8$)-Alkanoyloxy($C_1$-$C_{10}$)-alkyl steht.

4. Verfahren gemäß einem der Ansprüche 1 bis 3, dadurch gekennzeichnet, daß eine Verbindung der Formel I hergestellt wird, in welcher $R^4$ einen Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphtylalanin, 4-Nitrophenylalanin, Norleucin, 1,2,3,4-Tetrahydroisochinolin-3-carbonsäure, Homophenylalanin, 2-Amino-4-(2-thienyl)-buttersäure, (Z)-Dehydrophenylalanin der (E)-Dehydrophenylalanin bedeutet.

5. Verfahren gemäß einem der Ansprüche 1 bis 4, dadurch gekennzeichnet, daß eine Verbindung der Formel I hergestellt wird, in welcher B einen N-terminal mit A und C-terminal mit der NH-Gruppe von Formel I verknüpften Rest einer Aminosäure bedeutet, die wie die Aminosäuren in Anspruch 4 definiert ist.

6. Verfahren gemäß einem der Ansprüche 1 bis 5, dadurch gekennzeichnet, daß eine Verbindung der Formel I hergestellt wird, in welcher A einen N-terminal mit $R^1$ und C-terminal mit B verknüpften Rest einer Aminosäure bedeutet, die wie die Aminosäuren im Anspruch 4 definiert ist, (2S)-O-Benzyl-3-phenylpropanoyl oder (2R,S)-2-Benzyl-4oxo-6,6-dimethyl-6-heptanoyl bedeutet.

7. Verfahren gemäß einem der Ansprüche 1 bis 6, dadurch gekennzeichnet, daß eine Verbindung der Formel I hergestellt wird, in welcher $R^2$ Cyclohexylmethyl, Benzyl oder Isobutyl bedeutet.

8. Verfahren gemäß einem der Ansprüche 1 bis 7, dadurch gekennzeichnet, daß eine Verbindung der Formel I her gestellt wird, in welcher $R^3$ Wasserstoff, Ethyl, sec.-Butyl, Isobutyl, Benzyl, Phenethyl, oder 3-Phenylpropyl bedeutet.

9. Verfahren zur Herstellung eines pharmazeutischen Mittels enthaltend eine Verbindung, hergestellt gemäß einem der Ansprüche 1 bis 8, dadurch gekennzeichnet, daß man den Wirkstoff zusammen mit einem physiologisch annehmbaren Träger und gegebenenfalls weiterer Zusatz-oder Hilfsstoffe in eine geeignete Darreichungsform bringt.